# EUROPEAN PATENT SPECIFICATION

(11) **EP 3 022 785 B1**
(45) Date of publication and mention of the grant of the patent: **26.09.2018**
(21) Application number: 14747829.1
(22) Date of filing: 14.07.2014
(51) Int. Cl.: H01M 4/36, H01M 4/587, H01M 4/62, H01M 4/88, H01M 4/90, H01M 4/92, H01M 8/16, H01M 8/18, H01M 8/10

(54) **MICROTUBES MADE OF CARBON NANOTUBES**
MIKRORÖHRCHEN AUS KOHLENSTOFFNANORÖHREN
MICROTUBES CONSTITUÉS DE NANOTUBES DE CARBONE

(30) Priority: 16.07.2013 EP 13003572
(43) Date of publication of application: 25.05.2016
(73) Proprietor: DWI an der RWTH Aachen e.V., 52074 Aachen (DE)
(72) Inventor: GENDEL, Youri, 52134 Herzogenrath (DE); WESSLING, Matthias, 52074 Aachen (DE); DAVID, Oana, NL-6291 AH Vaals (NL)
(74) Representative: Müller-Boré & Partner Patentanwälte PartG mbB
(86) International application number: PCT/EP2014/001923
(87) International publication number: WO 2015/007382

(56) References cited:
- BR-A2- PI0 706 086
- US-A1- 2009 295 644

## Description

The present invention relates to the fabrication of microtubes made of carbon nanotubes or composites based on carbon nanotubes. The present disclosure also relates to the use of such microtubes made of carbon nanotubes or composites based of carbon nanotubes as stand alone electrodes or integrated with current collectors or as a part of membrane electrode assemblies applied in electrochemical systems such as primary and secondary batteries, redox flow batteries, fuel cells, electrochemical capacitors, capacitive deionization systems, electrochemical- and biosensors devices, or solar cells. Another use of the microtubes made of carbon nanotubes or composites based on carbon nanotubes relates to their application as supported or unsupported tubular membranes for water or wastewater filtration, in aqueous and organic solvent filtration, for blood filtration, for gas separation processes, in gas and liquid adsorption processes or in sensor applications.

Electrochemical systems such as fuel cells, electrochemical capacitors and redox flow batteries are recognized today as promising technologies for the electrical energy conversion and storage (EECS) that are required for the proper energy management of conventional power plants as well as for the effective utilization of a renewable energy obtained from solar radiation, wind power plants, renewable fuels, wave power and other sources. Tremendous afford is being made nowadays to develop economically feasible electrochemical alternatives for "conventional" electrical energy storage systems such as pumped hydroelectric storage (PHS), compressed air energy storage (CAES), flywheels and others. The sharp increase in the number of publications in the field of electrochemical electrical energy conversion and storage systems observed for the last seven years is a perfect indication of the importance of these systems in the nearest future.

The heart of the most electrochemical EECS reactors is the membrane electrode assembly (MEA). A typical MEA is comprised of two porous electrodes separated by the membrane intended to avoid direct contact of electrodes and to close the electrical circuit via selective or non-selective transport of ionic species.

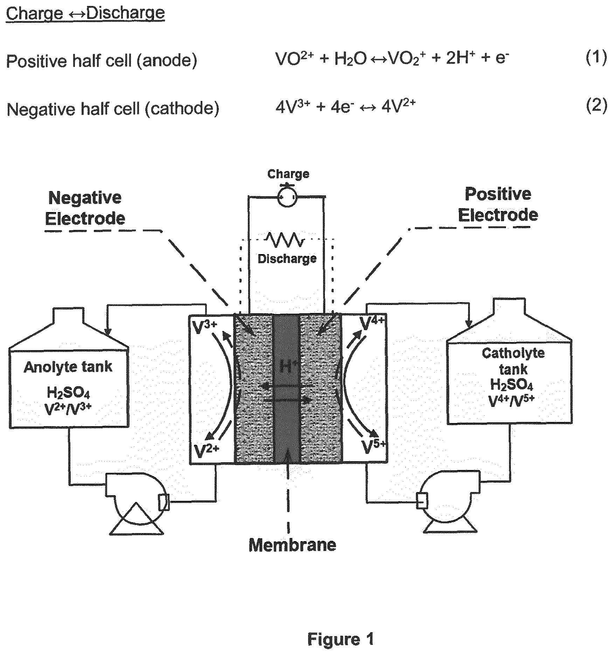

Oxidation and reduction processes occur on electrodes that are termed anode and cathode, respectively. Figure 1 shows the typical structure of an All-Vanadium Redox Flow Battery (AVRFB) to provide an example of EECS system that utilizes MEA. In AVRFB the MEA consists of two porous electrodes separated by a membrane. Electrolytes that store the chemical energy are aqueous solutions of sulfuric acid and V⁴⁺/V⁵⁺ and V²⁺/V³⁺ vanadium couples in positive and negative half cells, respectively. Chemical energy stored in AVRFB is converted into electrical energy (and *vice versa*) on porous electrodes while protons produced or consumed during the charge and discharge reactions (Eqs. 1-2) are selectively transported through the proton conductive membrane (PEM) typically of Nafion type or made of perfluorosulphonic acid or sulfonated polyarylethers.

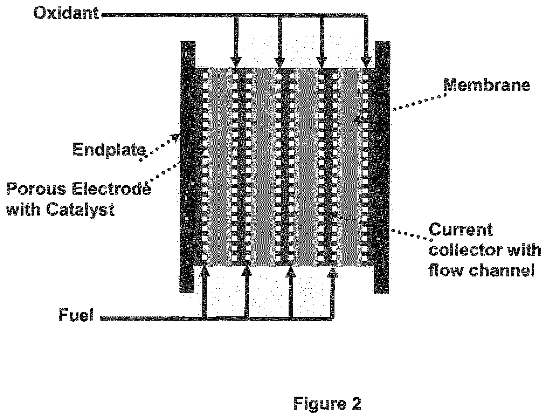

Usually electrochemical EECS systems like fuel cells and redox flow batteries are comprised of stacks of many MEAs with bipolar (or monopolar) electrodes separated by membranes (see Figure 2).

Electrodes applied in AVRFB are made of carbon, i.e. carbon cloth, felt, paper and others. The fact is that electrodes made of carbonaceous materials are used in most types of electrochemical EECS systems, such as hydrogen, methanol, ethanol, formic acid, ammonia, microbial and other fuel cells; lithium batteries; and electrochemical capacitors.

Unfortunately, full scale application of electrochemical EECS systems is still economically unfeasible. To overcome this obstacle, performance of electrochemical reactors has to be improved while the main goal is to increase power and/or energy densities of the system, which are two major parameters that characterize the performance of the electrochemical EECS system. The basic approach to improve the performance of the electrochemical EECS device is to increase the utilization efficiency of its components.

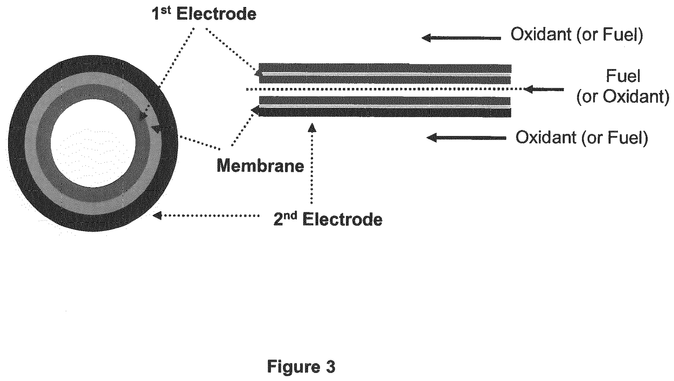

Better utilization of materials and higher power densities can be achieved *via* the improvement of the cell geometry. Similarly to the conventional planar membrane electrode assemblies used in redox flow batteries and fuel cells, a tubular MEA is also comprised of three basic layers: positive electrode, negative electrode and a membrane between the electrodes. The general structure of such tubular MEA is shown on Figure 3.

Tubular design is advantageous over the planar shape of electrochemical EECS systems due to three major reasons: tubular cells have higher power densities, lower manufacturing costs and lower parasitic power losses. These advantages are realized in solid oxide fuel cells (SOFC) where tubular geometry is common and prevails over the planar geometry. In fact, current research of SOFC is focused on micro-tubular cells with diameters of less than 2 mm.

Obviously, application of tubular MEAs would be also advantageous for most types of electrochemical EECS systems. Unfortunately, production of tubular fuel cells and redox flow batteries is hampered due to (apparently) unavailability of self-supporting, porous and tubular carbonaceous electrodes. In spite of this fact, the research aimed in the development of tubular electrochemical EECS systems is very intensive. Thus, the application of tubular design for proton exchange membrane fuel cells (PEMFC) has been suggested (Bullecks, B., Rengaswamy, R., Bhattacharyya, D., Campbell, 2011. Development of a cylindrical PEM fuel cell. International Journal of Hydrogen Energy. 36, 713-719). Here, a perforated plastic syringe was used to support the electrodes and the membrane. Moreover, there has been developed a tubular methanol fuel cell with a stainless steel tube as a MEA carrier. More tubular methanol fuel cells were proposed in the art with Flemion® tube as a carrier of MEA. There is also research aimed in realization of tubular design for microbial fuel cells (WO 2007/011206 A1).

Among numerous carbonaceous materials available today for the manufacture of electrochemical reactors, carbon nanotubes (CNT) play a special role due to the outstanding mechanical and electrochemical properties. Carbon nanotube is a pseudo one-dimensional material that can be considered as a cylinder made of rolled graphene sheet with the diameter of nanometer scale and length-to-diameter ratio of more than 1000. According to the number of graphitic layers, CNT can be classified as single walled (SWCNT), double walled (DWCNT), triple walled (TWCNT) and multi walled carbon nanotubes (MWCNT). SWCNT have outer and inner diameters of 1-3 nm and 0.4-2.4 nm, respectively. Outer diameter of MWCNT can be as low as 2 nm and up to 100 nm depending on the number of walls. Carbon-carbon sp² bonds in the CNT are much stronger than sp³ bonds in diamond structure. For that reason CNTs possess exceptional mechanical stability with Young's module as high as 1.2 TPa and tensile strength of 50-200 GPa. Electrical resistivities of SWCNT and MWCNT are about 10⁻⁶ and 3x10⁻⁵Ωcm, respectively, which makes them probably the best of known carbon made electrical conductors. Carbon nanotubes are recognized as superior catalysts carriers in proton exchanging membrane fuel cells (PEMFC) due to their surface properties and resistance to corrosion. In the state of the art, it is reported that acid treated MWCNT decorated with Pt showed four times higher durability than standard Pt/C catalysts. Effectiveness of Pt-CNT catalysts for oxygen reduction reaction in H₂-O₂ PEMFC was also proved. Moreover, carbon nanotubes loaded with Pt/IrO₂ have been successfully applied as anode catalyst for direct methanol fuel cell. Pd/SnO₂-TiO₂-MWCNT catalyst was found very effective for the direct formic acid fuel cell. Many other applications of CNT as a catalyst carrier in PEMFC can be found in relevant literature.

Carbon nanotubes can be assembled into macroscopic, freestanding films that are also called buckypapers (BP). These films are formed via the self assembly of CNT due to van-der-Waals forces in the tube-tube junctions, and can be prepared from both single walled and multi walled carbon nanotubes. Usually BP are manufactured *via* the filtration of suspension of CNT through a micrometer scale pore filter and subsequent washing and drying of the formed CNT mat. Evaporation of the solvent can also be applied for the production of the BP. Electrophoretic deposition is another technique that is applied for the preparation of free standing CNT films. In this case CNTs are positively or negatively charged in suspension before the electrophoretic deposition onto conductive or non-conductive supports. Depending on the preparation technique and applied conditions, BP with thicknesses of several microns and up to several hundreds of microns can be produced. CNT films can also be loaded with appropriate catalysts using elecrodepositon, electroless plating and other techniques. Alternatively, carbon nanotubes might be loaded with catalysts or modifiers prior to the fabrication of the buckypaper.

Buckypaper stand alone electrodes made of SWCNT and carbon nanofibers loaded with platinum catalysts were suggested for H₂-O₂ PEMFC and this material was found very effective. Moreover, SWCNT buckypaper loaded with platinum catalyst has been applied as a cathode for a microbial fuel cell. Further known are free-standing SWCNT buckypaper electrodes for lithium sulfur battery. Here, SWCNT applied for the preparation were filled with sulfur before the filtration of the suspension. A review article of Liu et al. provides details on numerous applications of CNT based composites in rechargeable Li-ion batteries (Liu, X. M., Huang, Z.D., Oh, S. W., Zhang, B., Ma, P.C., Yuen, M.M.F., Kim, J.K., 2012. Carbon nanotube (CNT)-based composites as electrode material for rechargeable Li-ion batteries: A review. Composites Science and Technology. 72, 121-144).

Due to high electrical conductivity, flexibility, mechanical stability, high surface area and high specific capacitance, buckypaper is an attractive electrode material in electrochemical capacitors (supercapacitors), capacitive deionization, and Capacitive Double Layer Expansion (CDLE) technologies applied for energy production from blending of waters with different salinities (also known as Blue Energy technologies). It has been shown that specific capacitance of DWCNT bucky paper was 32 F/g and this value was increased up to 129 F/g via electrodeposition of MnO₂ onto the BP. In fact, specific capacitance of CNT films can be improved by deposition of many types of redox-active metal oxides such as RuO₂, MoO₃, Ni(OH)₂, Co₃O₄, Fe₂O₃, In₂O₃, TiO₂, and V₂O₅.

Vanadium redox flow battery (RFB) is the most studied and promising type of RFB. Carbon nanotubes were also found an effective catalyst for the positive half cell reaction (V⁵⁺/V⁴⁺), while MWCNTs functionalized with carboxyl groups showed faster kinetics (about three times) than common electrode materials (Li, W., Liu, J., Yan, C., 2011, Multi-walled carbon nanotubes used as an electrode reaction catalyst for VO2+/VO2+ for a vanadium redox flow battery, Carbon, 49, 3463-3470).

Additional electrochemical applications of CNT and buckypapers known in the art include gas sensors and biosensors.

It can be concluded that CNT films might be successfully applied in any electrochemical Electrical Energy Conversion and Storage (EECS) systems where porous carbonaceous materials, such as carbon felt and cloth, are conventionally used due to better physico-chemical properties of CNT films.

Moreover, CNT films made of CNT can be applied for nanofiltration and ultrafiltration of waters and wastewaters, and for the gas separation processes as disclosed in: Sears, K., Dumee, L., Schutz, J., She, M., Huynh, C., Hawkins, S., Duke, M., Gray, S., 2010. Recent developments in carbon nanotube membranes for water purification and gas separation. Materials, 3, 127-149.

Additionally, carbon nanotubes and multi-walled CNT in particular are known to adsorb effectively carbon dioxide (Su, F., Lu, C., Chen, W., Bai, H., Hwang, J.F., "Capture of CO2 from flue gas via multiwalled carbon nanotubes", Science of the Total Environment, 2009, 407(8), 3017-3023). Performance of the CO₂ adsorption by MWCNT can be further improved via appropriate modifications of CNT (e.g by 3-aminopropyl-triethoxysilane).

Furthermore, carbon nanotubes in combination with ionic liquids are known to have potential applications as hybrid materials for gel electrodes, actuators, sensors and support for catalysts (Carbon nanomaterial - ionic liquid hybrids, M. Tunckol, J. Durand, P. Serp, Carbon, 50(4) 4303). The carbon nanotubes act as an electron conductor and simultaneously support and immobilize the ionic liquid into a paste which properties are influenced by the carbon nanotube content. The ionic liquid in turn acts as a solvent for heterogeneous or homogeneous chemical catalysts. Such pastes of CNT and ionic liquid are impossible to shape into a self-supporting microtube.

Finally, carbon surfaces are also known to selectively adsorb endotoxins from blood. It is desired to have a carbon surface in direct contact with blood to remove the toxin towards the membrane surface and have a low toxin concentration in the blood. This will cause further toxin decomplexation from the blood proteins. Blood treatment membranes are generally tubular and smooth surfaces are required. Embedding carbon particles into a polymer matrix often result in rough surfaces and smooth polymeric porous cover layers are used to prevent this problem. However, the adsorption layer lays underneath the cover layer. Endotoxins can only diffuse into the depth of the membrane body. Direct blood contact with a very smooth surface would be desirable.

In view of the above, the object underlying the present invention is to provide a new kind of material with microtubular geometry and outstanding porosity, mechanical and chemical stability, electrochemical properties, high electrical and thermal conductivity and high surface area and specific capacitance. Characteristics of the product should be adjustable to desired values using appropriate tuning and modifications of the production process. Outstanding properties should make such product highly valuable for electrochemical systems, thus being capable to be used both as a stand alone electrode and as a part of membrane electrode assemblies. Moreover, this new kind of material should be capable to be used for water or wastewater filtration, blood filtration, aqueous and organic solvent filtration or gas separation processes, for adsorption processes from a gaseous and liquid fluid.

According to the present invention, the above-described technical problem related to the production of tubular electrochemical reactors and filtration and/or adsorption devices is solved by providing stand alone (i.e. free-standing, self-supporting (unsupported), i.e. not requiring any support) microtubes made of carbon nanotubes or carbon nanotube based composites wherein the microtube has an outer diameter in the range of 500 to 5000 µm, particularly 1000 to 5000 µm, and a wall thickness in the range of 50 to 1000 µm, particularly 100 to 1000 µm.

Actually, the dimensions of the microtubes according to the present invention are in the millimetric range, i.e. bigger than to call them "microtubes". However, in publications relating to the present technical field, tubular electrodes with an outer diameter of less than 2 mm are termed "microtubular electrodes" (e.g. Howe, K.S., Gareth, J.T., Kendall, K., "Micro-tubular solid oxide fuel cells and stacks", Journal of Power Sources, 2011, 196(4), 1677-1686). Therefore, instead of "tubes", the term "microtubes" is used throughout the present specification.

The microtubes according to the present invention are comprised of carbon nanotubes or carbon nanotube based composites. Preferably, the microtubes consist of carbon nanotubes or carbon nanotube composites. In a preferred embodiment, the carbon nanotubes are multi-walled carbon nanotubes.

Preferably, the outer diameter of the stand alone microtubes is in the range of 1500 to 3000 µm, and the wall thickness is preferably in the range of 200 to 500 µm. The maximal length of the stand alone microtubes can be up to 200 cm, preferably the length of the microtubes is between 10 and 100 cm.

The microtubes according to the present invention can be formed in accordance to the desired geometry (outside and inside diameters and length), porosity, electrical conductivity and catalytic activity. The present invention further relates to the use of the microtubes made of carbon nanotubes or composites based on carbon nanotubes in electrochemical reactors as a stand alone, self-supporting electrode or/and as a part of a membrane electrode assembly.

Moreover, the present disclosure also relates to such microtubes with integrated current collector. CNT microtubes have a limited length in practical applications due to its limiting conductivity with length. This gradient in resistance causes a gradient in current over the length of CNT microtube electrodes. This resistance issue can be overcome by integrating the CNT microtube with a current collector, i.e. by providing CNT microtubes with in-wall current collectors, for example in the form of a spring made of e.g. titanium, copper, aluminum, titanium, platinum, nickel or stainless steel.

The present disclosure also relates to the use of the microtubes made of carbon nanotubes or composites based on carbon nanotubes as supported or unsupported tubular membranes, particularly for water or wastewater filtration, organic solvent filtration or gas separation processes. Supported microtubes can be of any thickness appropriate for the specific purpose.

The present disclosure further relates to the use of the supported or unsupported microtubes made of carbon nanotubes or composites based on carbon nanotubes with or without special modifications for the gas adsorption, in particular for CO₂ removal from flue gas or other types of gas. Another possible use is the use of the supported or unsupported microtubes made of carbon nanotubes or composites based on carbon nanotubes for electronic charge storage applications, in particular as supercapacitors.

BR PIO 706 086 relates to microtubes with an outer diameter of less than 20 microns in contrast to the tubes/microtubes according to the present invention that have outer diameter of up to in the range of 500 to 5000 µm. Moreover, in BR PIO 706 086 wall thicknesses are less than 1 µm, while in the tubes/microtubes according to the present invention minimal wall thickness is 100 µm. Due to the small size, the intended applications as addressed above (fabrication of tubular electrochemical cells and watergas separation process where primary gas or liquid phase is flowed inside the tube and secondary gaseous or liquid phase is located outside the tube, microtubes with in-wall current collectors) are impossible for the microtubes disclosed in said BR PIO 706 086.

The further figures are as follows:

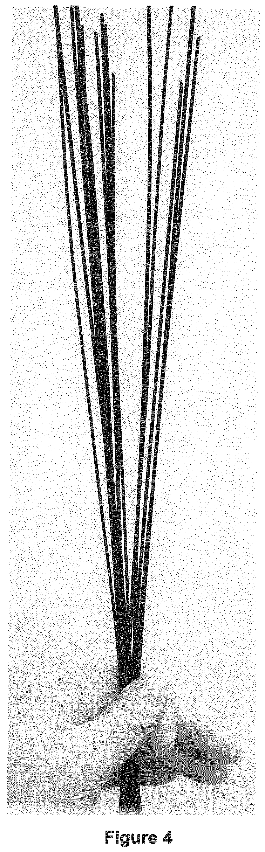

**Figure 4** shows the microtubes made of multi-walled carbon nanotubes prepared using the filtration of MWCNT suspension through the microfiltration hollow fiber polypropylene membrane.

**Figure 5** represents the variation of pressure applied by the syringe pump during the filtration of MWCNT suspension through the polypropylene microfiltration hollow fiber membranes. Two repetitions at identical conditions are shown at constant flow rate of 1 ml/min, and suspension made of 1 g/l of multi-walled carbon nanotubes and 10 g/l of Triton X-100 as a dispersant in distilled water.

**Figure 6** shows the final stage of preparation of stand-alone microtube made of MWCNT while it is being withdrawn from the polypropylene hollow fiber membrane applied for the preparation.

**Figure 7** shows results of thermogravimetric analysis obtained for different volumes of isopropanol applied for the removal of Triton X-100 surfactant used for the preparation of CNT suspension. Two repetitions for each load of 2-propanol are shown. Microtubes made at MWCNT loads of 8.04 mg/cm² were analyzed.

**Figure 8** shows the scanning electron microscope images(SEM) (magnification of 50) of MWCNT-microtubes prepared with MWCNT loadings of 8.04 mg/cm² (**8a**) and 4.01 mg/cm² (**8b**).

**Figure 9** shows the SEM images (magnification of 50) of cross sections of MWCNT-microtubes prepared with MWCNT loadings of 8.04 mg/cm² (**9a**), 6.03 mg/cm² (**9b**) and 4.01 mg/cm² (**9c**).

**Figure 10** shows the SEM images (magnifications ef 50 and 500) of the cross section of the membrane electrode assembly prepared from microtube made of multi-walled carbon nanotubes and Nafion-117 proton exchanging membrane.

**Figure 11** shows the membrane electrode assembly comprised of microtubes made of carbon nanotubes and porous polypropylene membrane.

**Figure 12** shows CNT microtubes with in-wall current collectors and their preparation.

The present disclosure relates to the microtubes made of carbon nanontubes or composites based on carbon nanotubes and its applications for the manufacture of electrochemical reactors as a stand alone electrodes or/and as a part of membrane electrode assemblies.

The present disclosure also relates to microtubes made of carbon nanotubes with layered structure, which means that the wall of the microtubes is comprised of different layers of materials such as modified and not modified carbon nanotubes, single walled and multi walled carbon nanotubes, layers of additives such as catalysts, polymers, carbon nanofibers or other nanosize materials and other types of materials desired for the specific application.

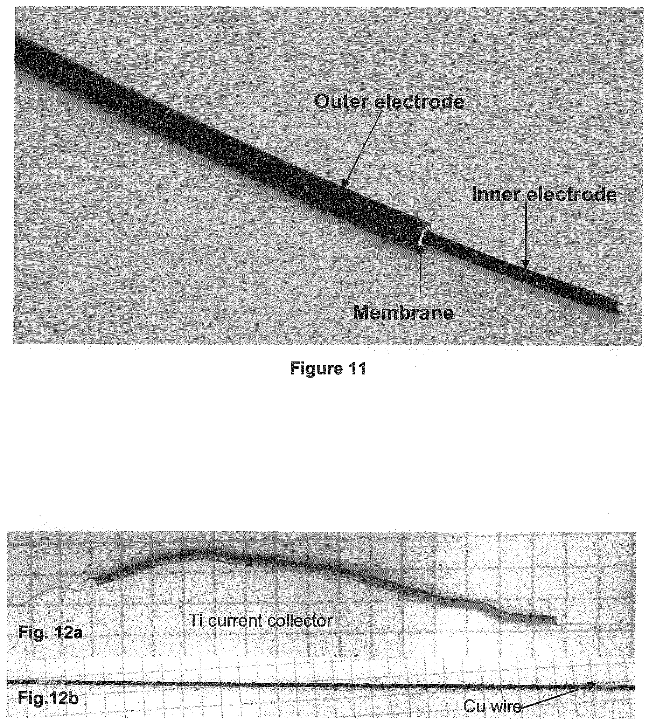

Membrane electrode assemblies according to the present disclosure are:
1. Membrane electrode assembly comprised of microtube made of carbon nanotubes and coated with porous or non-porous, selective or not selective membrane on the interior or exterior surface; and
2. Membrane electrode assembly comprised of two or more microtubes made of carbon nanotubes located one inside another with the porous or non-porous membranes between them (cf. Figure 11).

Electrochemical reactors according to the present disclosure are: primary and secondary batteries, fuel cells, redox flow batteries, electrochemical capacitors, gas-, bio- and other sensors, microbial fuel cells and solar cells.

According to the present invention the microtubes can be manufactured from any type of carbon nanotubes, i.e. single walled and multi-walled or their mixtures. Additives might be used for the preparation of microtubes with specific electrochemical or physical properties, in this case the microtubes are termed "microtubes made of carbon nanotubes based composites". Thus, microtubes with specific catalytic activity are produced from carbon nanotubes loaded with desired catalysts (in particular metals, salts of metals or/and their oxides). Modifiers can be loaded to the carbon nanotubes prior to the fabrication of the microtubes to alter their surface area and specific capacitance, several examples of this modifiers are: RuO₂, MoO₃, Ni(OH)₂, Co₃O₄, Fe₂O₃, In₂O₃, TiO₂, and V₂O₅. Carbon nanotubes with altered surface chemistry (for example carbon nanotubes functionalized with (-COOH), hydroxyl (-OH) and carbonyl (-C=O) groups) are applied for the fabrication of microtubes with specific physico-chemical and catalytic properties.

Where composites based on carbon nanotubes are adopted, the nanotubes can further comprise materials dense or porous nanometer-sized particles selected from the classes of metals, metal oxides, metal organic frameworks and zeolites. In an embodiment of the present invention, the nanotubes can further comprise materials selected from the group consisting of LiCoO₂, LiMnO₂, LiNiO₂, LiMn₂O₄, Li(Ni_{1/2}Mn_{1/2})O₂, LiFePO₄, conductive polymers, Li₄Ti₅O₁₂, transitional metal oxides, TiO₂, SnO₂, Si, and sulfur. In a further embodiment, the composites based on the carbon nanotubes further comprise carbon based particles selected from the class of graphenes, nanoribbons, carbon-like dendrimers and carbon nanoparticles. Microtubes made of carbon nanotube based composites can be fabricated and used as a carrier of the electrochemically active material. For example, the present invention also relates to tubular lithium ions batteries fabricated from microtubes made of carbon nanotubes based composites with the following cathode materials: LiCoO₂, LiMnO₂, LiNiO₂, LiMn₂O₄, Li(Ni_{1/2}Mn_{1/2})O₂ and many others.

Two methods are appropriate for the fabrication of microtubes according to the present invention:
1. Filtration of a suspension of carbon nanotubes through an appropriate porous membrane of tubular form; and
2. Electrophoretic deposition of carbon nanotubes from a suspension of carbon nanotubes onto a carrier of tubular form.

Preparation of a suspension of carbon nanotubes is well known to a person skilled in the art and numerous procedures are appropriate for the fabrication of CNT suspensions suitable of the manufacture of microtubes made of carbon nanotubes. In general, the preparation of CNT suspension is comprised of several major steps that are briefly described here. Prior to the preparation of the suspension, CNT are pretreated to remove carbonaceous impurities and residuals of catalysts used within the preparation of nanotubes (Fe, Co, Ni, Au, Pd, Ag, Pb, Mn, Cr, Ru, Mo, Cu). Next, modification of carbon nanotubes described previously can be done. This step is followed by the preparation of the suspension. Numerous solvents are known for the preparation of the CNT suspensions. A few examples are: isopropyl alcohol (IPA), N-methylpyrrolidone (NMP), N,N-dimethylformamide (DMF) and water. CNT suspensions in water are prepared using a suitable surfactant such as Triton X-100, sodium dodecylbenzene sulfonate (NaDDBS), sodium dodecyl sulfate (SDS), dihexadecyl hydrogen phosphate or other compounds. Carbon nanotubes charged with positive and negative groups can be applied for the preparation of CNT suspension without application of surfactants or other dispersants. Additionally, suspensions of carbon nanotubes applied for the microtubes according to the present invention can be comprised of mixtures of carbon nanotubes and other organic and inorganic materials, such as carbon nanofibers, polymers, metals, and so on. Ultrasonic treatment is usually applied as a final step for the preparation of the CNT suspension.

According to the present invention, the fabrication of the microtubes *via* filtration can be carried out as follows: The suspension of CNTs is filtered through a tubular porous membrane. Ultrafiltration and microfiltration tubular, hollow fiber membranes made of ceramics or polymeric materials, particularly polypropylene, can be used for the preparation of microtubes made of carbon nanotubes and CNT based composites according to the present invention. Such ultrafiltration (UF) and microfiltration (MF) hollow fiber membranes are commercially available (e.g. from Spectrum Labs) in different geometrical sizes (most common outer and inner diameters of 1 to 10 mm and 0.5 to 8 mm, respectively) and different pore size distributions. Typical UF and MF membranes have inner diameters of 0.5 to 8 mm and are available within a wide range of characteristics.

The final length and inside/outside diameters of the microtubes depend on the geometry of the membrane, properties of the carbon nanotubes, composition of the suspension and the volumetric load of the suspension on the membrane applied for the filtration. Filtration can be performed in both inside → outside and outside → inside directions. Membranes with micro- and nanostructured surface textures can be applied for the fabrication of micro- and nanostructured microtubes made of carbon nanotubes and carbon nanotubes based composites.

Secondly, fabrication of microtubes made of carbon nanotubes or carbon nanotubes based composites *via* the electrophoretic deposition can be carried out on porous and non-porous, electrically conductive and non-conductive support material of tubular form. Electrophoretic deposition can be performed on the inner or outer surface of tubular support using one outer cylindrical electrode with the support located inside thereof and the secondary electrode located inside the tubular support. Depending on the charge (positive or negative) of the carbon nanotubes and the desired surface of coating (internal or external) of the tubular support, an electrical field of appropriate direction and appropriate intensity is applied between two auxiliary electrodes via their polarization for the desired period of time. In case of electrically conductive support it can be used as an auxiliary electrode during the electrophoretic deposition of carbon nanotubes.

Microtubes made of carbon nanotubes with aligned carbon nanotubes can be prepared using known techniques. For example, application of a magnetic field during the filtration process can be used for the alignment of carbon nanotubes in the microtube made of carbon nanotubes or composites based on carbon nanotubes.

Next step of the fabrication of the microtubes made of carbon nanotubes can be the removal of dispersing agents (if applied). This is done by washing of the support loaded with carbon nanotubes with appropriate liquid. For example, if the filtration method is applied for the fabrication of microtubes made of carbon nanotubes, isopropanol can be filtrated through the porous support after the infiltration of CNT suspension in the same direction of filtration until the complete removal of the surfactant.

The last general step of the fabrication of microtubes made of carbon nanotubes (and their composites) is the drying step. This is usually done by using vacuum or air or inert atmosphere at different temperatures until the desired purity of the product is achieved. Due to the shrinking of the CNT film during drying, the resulting microtube can easily be removed from the support applied for its preparation. Alternatively, the composite (microtube made of carbon nanotubes and the support applied for the preparation) is a product itself.

Preparation of membrane electrode assemblies can be done *via* formation of microtubes on one or both surfaces of the tubular support using filtration method or electrophoretic deposition or both.

Preparation of membrane electrode assemblies based on microtubes made of carbon nanotubes or composites based on carbon nanotubes and a porous membrane can be done *via* the filtration of CNT suspension in the inside → ouside direction to form the internal microtubular electrode followed by the washing, drying and removal of the internal electrode. Next, the secondary electrode is applied to the outer surface of the porous tubular support. A microtubular CNT electrode is formed on the outer surface of the porous support using the outside → inside filtration of the CNT suspension. After the washing and drying of the outer microtube made of carbon nanotubes, the inner electrode is placed back into the porous support and the fabrication of MEA is finished; cf. also Figure 11.

The fabrication of membrane electrode assemblies based on non-porous membranes can be performed using preparation of free standing microtube made of carbon nanotubes using the filtration method. Next, a membrane is formed on the outer surface of the microtube. For the ion conductive membranes, casting of a monomer solution with subsequent heat curing can be applied. Alternatively, the microtube is inserted into a tubular membrane of appropriate geometry and nature. To accomplish the fabrication of MEA, the first tube with the coated membrane is inserted into the secondary electrode which is another microtube made of carbon nanotube of the appropriate geometry, or other tubular electrode. Alternatively, the secondary microtubular electrode made of CNT (or its composites) can be applied by using the electrophoretic deposition method.

The present invention will now be further illustrated in the following examples without being limited thereto

### EXAMPLES

### Example 1: Free standing microtubes made of multi-walled carbon nanotubes

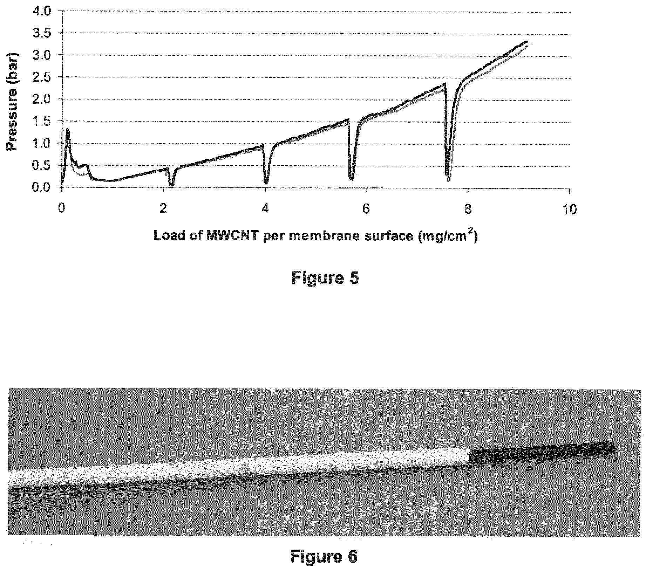

Multi walled carbon nanotubes (MWCNT) (> 95% purity, Sigma-Aldrich) with outer diameter of 6-9 nm and 5 µm length were used as received, without any pretreatment. Water suspension of pristine CNT was prepared as following: 1 gram of CNT was mixed with 10 g of Triton-X 100 (laboratory grade, Sigma-Aldrich) surfactant in 1 liter of distilled water (18 mΩ), magnetically stirred for 30 minutes and sonicated for 3 h (75 % amplitude, UP200S, Hielscher) in 1 liter Duran bottle that was immersed into the ice bath to prevent overheating of the suspension. Microtubes made of MWCNT (Figure 5) were prepared using the inside → outside filtrations of MWCNT suspensions through the polypropylene (PP) microfiltration (MF) hollow fiber membranes (PP S6/2, Accurel) of 46.5 cm length and 1.8±0.15 and 0.45±0.05 mm inside diameter and the wall thickness, respectively. One end of each hollow fiber was sealed with adhesive glue and the suspension of MWCNT was supplied into the open end of the membrane with the syringe pump (PHD Ultra, Harvard apparatus) equipped with 50 ml plastic syringe at 1 ml/min flow rate. Filtrated solution was collected into the graduated cylinder. Active length of MF membranes available for filtration was 44 cm. Five types of microtubes were prepared within this study, while different volumes of filtrated MWCNT suspensions were applied for each type of microtubes: 100, 125, 150, 175 and 200 ml, which correspond to 4.01, 5.02, 6.03, 7.034, and 8.04 mg/cm² of MWCNT loads onto the inside surface of the PP membrane (respectively). The length of resulting microtube was close to 44 cm. Next, isopropanol (Applichem, 98% purity) was filtrated through the CNT loaded hollow fibers (in-->out) to remove the surfactant and dried overnight in the vacuum oven at 30°C.

After the drying MWCNT-microtubes were removed from the polypropylene (PP) hollow fiber support, microtubes were stored in the vacuum oven at 30°C before further analysis.

Figure 5 represents the pressure increase measured with pressure transmitter (P-31, Wika, Germany) during the filtration of MWCNT suspension through the MF membranes. These membranes have a burst and implosion pressures of ≥ 4 and ≥ 8 bars, respectively. According to the Figure 5 pressures applied for the preparation of MWCNT-microtubes were inside the safe range (less than 3 bars). Pressure drops that appear on Figure 5 are due to periodical refilling of the syringe with new portions of MWCNT suspension. Figure 6 illustrates the MWCNT-microtube while removed out from the PP MF hollow fiber membrane.

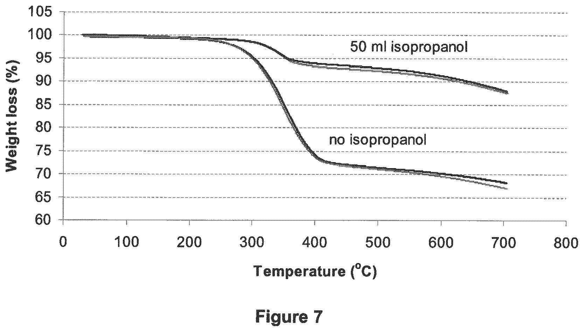

Effectiveness of the dispersing agent (Triton X-100 in this case) removal was studied using the TGA analysis. Three types of MWCNT were analyzed with two repetitions for each of them: microtubes prepared without isopropanol washing; washed with 50 ml of isopropanol; and with 100 ml of isopropanol. According to Figure 7, the weight loss starting at about 270°C occurs due to the evaporation of Triton-X 100 (b.p 270°C). Almost complete removal of the surfactant can be achieved by application of 100 ml of isopropanol, alternatively heat treatment in vacuum (or inert atmosphere) at about 300°C can be applied. Microtubes made without the washing step contained a lot of defects, for this reason application of washing is always needed.

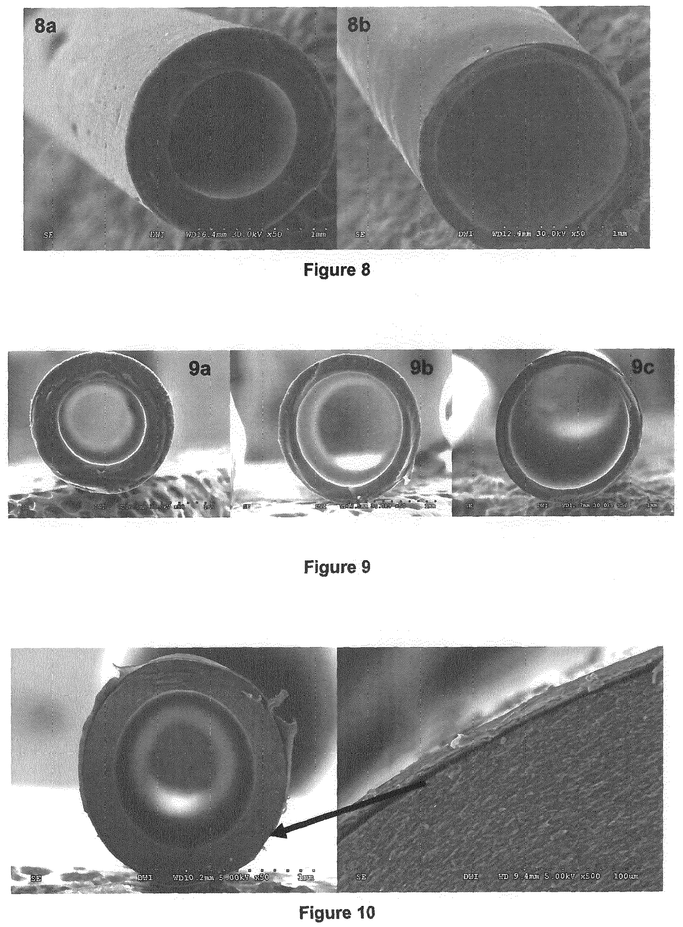

Figures 8 and 9 represent SEM (Hitachi S-3000N) images of MWCNT-microtubes. Cross section views, like those shown on Figure 9, were used to determine wall thicknesses and diameters of the MWCNT-microtubes.

Table 1 lists the physical properties of the manufactured MWCNT-microtubes. Average density, porosity and pore width of the MWCNT microtubes' walls are 360 ± 15.3mg/cm³, 58 ± 7.2 % and 25 ± 2.9 nm respectively. BET surface area, porosity and pore width of MWCNT-microtubes were determined with ASAP 2020 (Micromeritics) apparatus.

**Table 1. Physical properties of MWCNT-microtubes. Values of standard deviations (%) appear in brackets.**

| **Load of MWCNT** | **Outer radius** | **Wall thickness** | **Cross section area** | **Density** | **BET surface area** | **Porosity** | **Average Pore width** |
|---|---|---|---|---|---|---|---|
| (mg/cm²) | (µm) | (µm) | (mm²) | (mg/cm³) | (m²/g) | (%) | nm |
| 4.01 | 856 (0.33) | 134.8 (4) | 0.668 (3.74) | 367 (4.42) | 198.63 | 60.62 | 26.7 |
| 5.02 | 870.3 (0.7) | 180.3 (4.7) | 0.883 (0.96) | 342.9 (9.32) | 218.33 | 48.82 | 22.8 |
| 6.03 | 848.8 (0.7) | 208.8 (2.5) | 0.976 (2.76) | 360 (2.72) | 234.14 | 51.54 | 21.3 |
| 7.03 | 836.2 (0.7) | 274.4 (3.6) | 1.205 (3.71) | 352 (3.66) | 225.34 | 63.2 | 27.6 |
| 8.04 | 830 (1.62) | 314.3 (4.4) | 1.329 (5.37) | 383 (5.25) | 209.63 | 67.6 | 27.43 |

Table 2 concentrates the data concerning the electroconductivity/resistivity of MWCNT-microtubes prepared during this study. Electrical conductivities of the microtubes were measured using the 4 probes method with potentiostat/galvanostat (Autolab, PGSTAT302N, Metrohm).

**Table 2. Electrical conductivity of MWCNT-microtubes (in brackets: standard deviations in %).**

| **Load** | **Resistance** | **Resistivity** | **Conductivity** |
|---|---|---|---|
| (mg/cm²) | (ohm/cm) | (Ohm·cm) | (S/cm) |
| 4.01 | 6.42 (2.16) | 0.0428 (3.73) | 23.36 (3.96) |
| 5.02 | 5.64 (5) | 0.0498 (0.96) | 20.2 (0.89) |
| 6.03 | 4.58 (3.67) | 0.0447 (2.05) | 23.96 (2.23) |
| 7.03 | 3.86 (2.74) | 0.0465 (3.71) | 21.36 (3.29) |
| 8.04 | 3.09 (6.97) | 0.0411 (5.38) | 24.36 (5.25) |

### Example 2: Membrane electrode assembly with proton exchanging membrane and microtube made of carbon nanotubes

Microtubes made of multi-walled carbon nanotubes with outer radius of 836 (±0.7%) µm and the wall thickness of 274.4 (± 3.6%) µm were coated with the Nafion 117 solution (5%, Aldrich) by brushing and air dried. Heat curing was performed in the vacuum oven at 150°C for 6 hours. Figures 11 shows the cross section images of the membrane electrode assembly recorded using the scanning electron microscope.

Resulting thickness of the membrane was approximately 15 µm. The MEA was tested for leakages with 5 M sulfuric acid that was pumped through the tubular MEA at the flow rate of 30 ml/min using the peristaltic pump. No visible losses of solution through the MEA were detected. This membrane electrode assembly can be assembled with the secondary microtubular CNT electrode using the electrophoretic deposition method. Alternatively, separately manufactured microtube made of carbon nanotubes might be assembled as an outer electrode for the fabrication of MEA. Membrane electrode assembly disclosed in this section can be used for the most type of proton exchanging fuel cells, all vanadium redox flow battery and other electrochemical systems for conversion and storage of electrical energy.

### Example 3: Tubular MEA comprised of microtubes made of carbon nanotubes and polypropylene microfiltration membrane

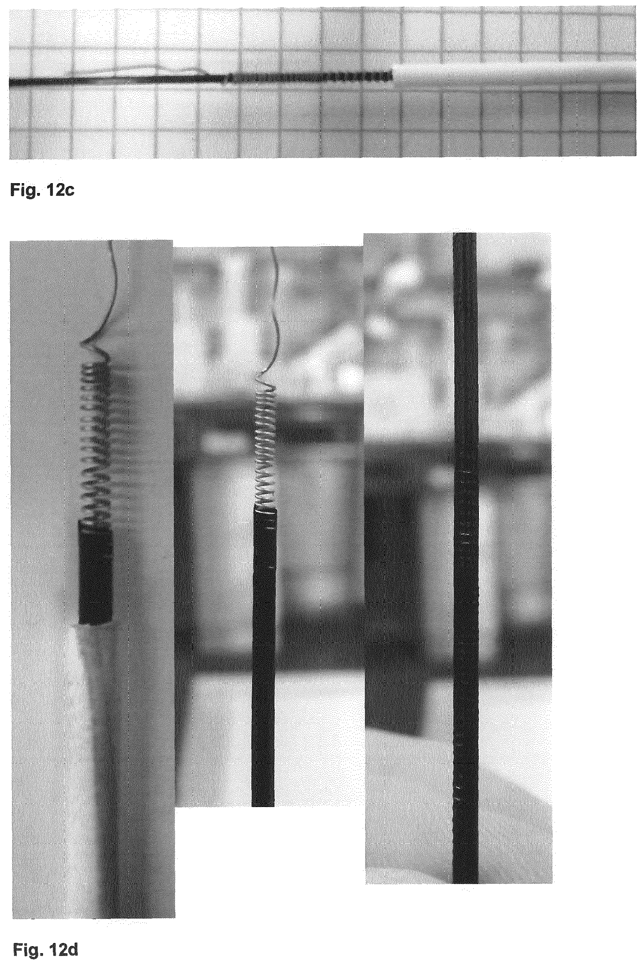

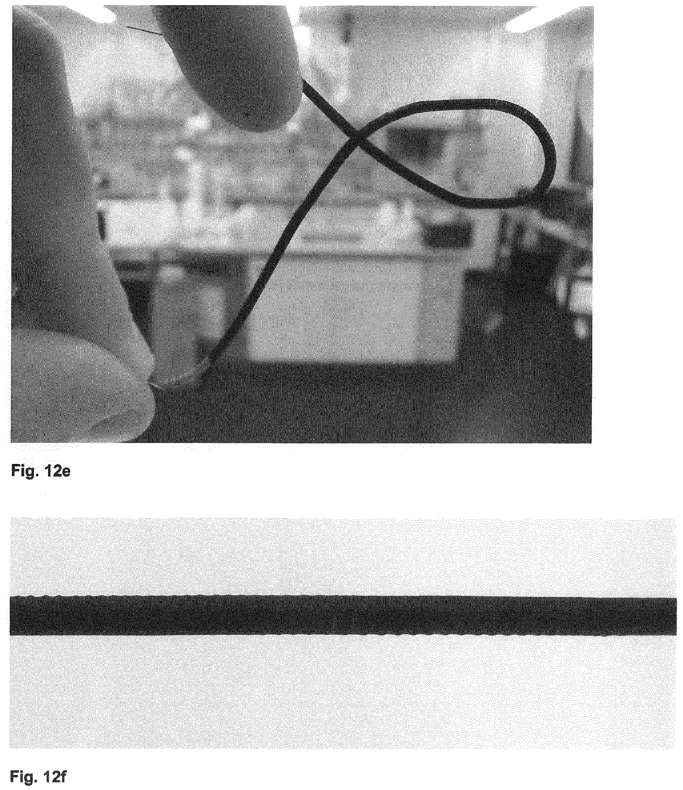

175 ml of suspension of multi-walled carbon nanotubes were filtered through the polypropylene microfiltration membrane (inside → outside), washed with 100 ml of isopropanol and dried in vacuum oven for 24 hours at 30°C (for the details, please see Example 1). Afterwards the microtube was removed from the polypropylene support and the microfiltration membrane was used again to prepare the secondary microtube made of carbon nanotubes on the outer surface of the membrane. This time 300 ml of suspension of CNT were filtered through the membrane in the outside → inside direction, washed with 100 ml of isopropanol and dried at vacuum oven. Finally, inner electrode was inserted into the hollow fiber to accomplish the MEA. Figure 12 shows the resulting membrane electrode assembly. This type of MEA with porous membrane can be applied for electrochemical reactors where mixing of catholyte and anolyte is allowed. One example of such application is a microbial fuel cell.

### Example 4: CNT microtubes with in-wall current collectors

### 1. Materials

### Current collector materials:

1) Titanium wire 0.2 mm diameter
2) Copper wire 0.18 mm diameter

### CNT suspension:

1 g/l water suspension of MWCNT and 10g/I Triton X-100.

### Membrane:

Microfiltration hollow fiber membrane (PP S6/2, Accurel) of 11, 15, 16 and 17.5 cm lengths and 1.8±0.15 and 0.45±0.05 mm inside diameter and the wall thickness, respectively

### 2. Preparation

Ti and copper collectors in the form of springs (Fig. 12a) were made using a 1 mm diameter rod (Fig.12b). 1. The titanium current collector (Fig. 12a) and copper current collector (Fig. 12b) were rolled over 1 mm supporting rods. Next, the current collectors were inserted into the MF hollow fiber membranes and the supporting rods were removed. Fig. 12c shows the titanium current collector inserted into the MF hollow fiber membrane. Then, one end of the hollow fiber was sealed with adhesive glue and the MWCNT suspension was infiltrated (in--> out) through the membrane at a flow rate of 1 ml/min to form a CNT-microtube with a wall thickness of about 180 µm. Afterwards, Triton X-100 was removed via infiltration of isopropanol (50 ml). Finally, the tubes were dried in the vacuum oven at 30°C.

### 3. Results

Figures 12d-f show CNT microtubes with Ti current collectors.

## Claims

1. A process for fabricating a stand alone, self-supporting microtube made of carbon nanotubes or composites based on carbon nanotubes, wherein the microtube has an outer diameter in the range of 500 to 5000 µm and a wall thickness in the range of 50 to 1000 µm, comprising either
filtration of a suspension of carbon nanotubes through an porous membrane of tubular form; or
electrophoretic deposition of carbon nanotubes from a suspension of carbon nanotubes onto a carrier of tubular form.

2. The process according to claim 1, wherein the microtube has an outer diameter in the range of 1500 to 3000 µm and a wall thickness in the range of 200 to 500 µm.

3. The process according to claim 1 or 2, which has a maximal length of up to 200 cm, preferably a length in the range of 10 to 100 cm.

4. The process according to any one of claims 1 to 3, wherein the carbon nanotubes are multi walled carbon nanotubes.

5. The process according to any one of claims 1 to 3, wherein the carbon nanotubes are single walled carbon nanotubes.

6. The process according to any one of claims 1 to 5, wherein the carbon nanotubes are loaded with catalysts or modifiers.

7. The process according to any one of claims 1 to 6, wherein the carbon nanotubes are functionalized with (-COOH), hydroxyl (-OH) and carbonyl (-C=O) groups.

8. The process according to any one of claims 1 to 7, wherein the carbon nanotubes are aligned.

9. The process according to any one of claims 1 to 8, wherein the composites based on the carbon nanotubes further comprise dense or porous nanometer-sized particles selected from the classes of metals, metal oxides, metal organic frameworks and zeolites.

10. The process according to any one of claims 1 to 9, wherein the composites based on the carbon nanotubes further comprise carbon based particles selected from the class of graphenes, nanoribbons, carbon-like dendrimers and carbon nanoparticles.

11. The process according to any one of claims 1 to 10, wherein the composites based on carbon nanotubes further comprise materials selected from the group consisting of LiCoO₂, LiMnO₂, LiNiO₂, LiMn₂O₄, Li(Ni_{1/2}Mn_{1/2})O₂, LiFePO₄, conductive polymers, Li₄Ti₅O₁₂, transitional metal oxides, TiO₂, SnO₂, Si, and sulfur.

12. The process according to any one of claims 1 to 11, wherein the carbon nanotubes are loaded with catalysts or modifiers prior to the fabrication of the microtubes.

13. The process according to any one of claims 1 to 11, wherein the carbon nanotubes are loaded with catalysts or modifiers after or during the fabrication of the microtubes.

14. The process according to any one of claims 1 to 13, further including the step of drying by using vacuum, air or inert atmosphere.

## Patentansprüche

1. Verfahren zur Herstellung einer freistehenden selbst-stützenden Mikroröhre, hergestellt aus Kohlenstoffnanoröhrchen oder Kompositen basierend auf Kohlenstoffnanoröhrchen, wobei die Mikroröhre einen äußeren Durchmesser in dem Bereich von 500 bis 5000 µm und eine Wanddicke in dem Bereich von 50 bis 1000 µm aufweist, umfassend entweder
Filtration einer Suspension von Kohlenstoffnanoröhren durch eine poröse Membran von tubulärer Form, oder
elektrophoretische Abscheidung von Kohlenstoffnanoröhren aus einer Suspension von Kohlenstoffnanoröhren auf einen Träger von tubulärer Form.

2. Verfahren gemäß Anspruch 1, wobei die Mikroröhre einen äußeren Durchmesser in dem Bereich von 1500 bis 3000 µm und eine Wanddicke in dem Bereich von 200 bis 500 µm aufweist.

3. Verfahren gemäß Anspruch 1 oder 2, wobei die Mikroröhre eine maximale Länge von bis zu 200 cm, vorzugsweise eine Länge in dem Bereich von 10 bis 100 cm aufweist.

4. Verfahren gemäß einem der Ansprüche 1 bis 3, wobei die Kohlenstoffnanoröhren mehrwändige Kohlenstoffnanoröhren sind.

5. Verfahren gemäß einem der Ansprüche 1 bis 3, wobei die Kohlenstoffnanoröhren einzelwändige Kohlenstoffnanoröhren sind.

6. Verfahren gemäß einem der Ansprüche 1 bis 5, wobei die Kohlenstoffnanoröhren mit Katalysatoren oder Modifiziermitteln beladen sind.

7. Verfahren gemäß einem der Ansprüche 1 bis 6, wobei die Kohlenstoffnanoröhren mit (-COOH), Hydroxyl (-OH) und Carbonyl (-C=O) Gruppen funktionalisiert sind.

8. Verfahren gemäß einem der Ansprüche 1 bis 7, wobei die Kohlenstoffnanoröhren ausgerichtet sind.

9. Verfahren gemäß einem der Ansprüche 1 bis 8, wobei die Komposite basierend auf den Kohlenstoffnanoröhren weiter dichte oder poröse nanometer-große Teilchen, ausgewählt aus der Klasse von Metallen, Metalloxiden, metallorganischen Gittergerüsten und Zeolithen, umfassen.

10. Verfahren gemäß einem der Ansprüche 1 bis 9, wobei die Komposite basierend auf den Kohlenstoffnanoröhren weiter Teilchen auf Kohlenstoff-Basis, ausgewählt aus der Klasse von Graphenen, Nanoribbons, kohlenstoffartigen Dendrimeren und Kohlenstoffnanoteilchen, umfassen.

11. Verfahren gemäß einem der Ansprüche 1 bis 10, wobei die Komposite basierend auf Kohlenstoffnanoröhren weiter Materialien umfassen, ausgewählt aus der Gruppe, bestehend aus LiCoO₂, LiMnO₂, LiNiO₂, LiMn₂O₄, Li(Ni_{1/2}Mn_{1/2})O₂, LiFePO₄, leitfähigen Polymeren, Li₄Ti₅O₁₂, Übergangsmetalloxiden, TiO₂, SnO₂, Si und Schwefel.

12. Verfahren gemäß einem der Ansprüche 1 bis 11, wobei die Kohlenstoffnanoröhren mit Katalysatoren oder Modifiziermitteln vor der Herstellung der Mikroröhren beladen werden.

13. Verfahren gemäß einem der Ansprüche 1 bis 11, wobei die Kohlenstoffnanoröhren mit Katalysatoren oder Modifiziermitteln nach oder während der Herstellung der Mikroröhren beladen werden.

14. Verfahren gemäß einem der Ansprüche 1 bis 13, weiter einschließend den Schritt des Trocknens unter Verwendung von Vakuum, Luft oder inerter Atmosphäre.

## Revendications

1. Procédé de fabrication d'un microtube autonome autoportant réalisé en nanotubes de carbone ou composites basés sur des nanotubes de carbone, dans lequel le microtube a un diamètre externe dans la plage de 500 à 5000 µm et une épaisseur de paroi dans la plage de 50 à 1000 µm, comprenant soit
la filtration d'une suspension de nanotubes de carbone à travers une membrane poreuse de forme tubulaire ; soit
le dépôt électrophorétique de nanotubes de carbone provenant d'une suspension de nanotubes de carbone sur un support de forme tubulaire.

2. Procédé selon la revendication 1, dans lequel le microtube a un diamètre externe dans la plage de 1500 à 3000 µm et une épaisseur de paroi dans la plage de 200 à 500 µm.

3. Procédé selon la revendication 1 ou 2, qui a une longueur maximale allant jusqu'à 200 cm, de préférence une longueur dans la plage de 10 à 100 cm.

4. Procédé selon l'une quelconque des revendications 1 à 3, dans lequel les nanotubes de carbone sont des nanotubes de carbone à parois multiples.

5. Procédé selon l'une quelconque des revendications 1 à 3, dans lequel les nanotubes de carbone sont des nanotubes de carbone à paroi unique.

6. Procédé selon l'une quelconque des revendications 1 à 5, dans lequel les nanotubes de carbone sont chargés de catalyseurs ou modificateurs.

7. Procédé selon l'une quelconque des revendications 1 à 6, dans lequel les nanotubes de carbone sont fonctionnalisés avec des groupes (-COOH), hydroxyle (-OH) et carbonyle (-C=O).

8. Procédé selon l'une quelconque des revendications 1 à 7, dans lequel les nanotubes de carbone sont alignés.

9. Procédé selon l'une quelconque des revendications 1 à 8, dans lequel les composites basés sur les nanotubes de carbone comprennent en outre des particules de taille nanométrique denses ou poreuses sélectionnées parmi les classes des métaux, oxydes métalliques, ossatures organiques métalliques et zéolites.

10. Procédé selon l'une quelconque des revendications 1 à 9, dans lequel les composites basés sur les nanotubes de carbone comprennent en outre des particules à base de carbone sélectionnées parmi la classe des graphènes, nanorubans, dendrimères de type carbone et nanoparticules de carbone.

11. Procédé selon l'une quelconque des revendications 1 à 10, dans lequel les composites basés sur des nanotubes de carbone comprennent en outre des matériaux sélectionnés parmi le groupe constitué de LiCoO₂, LiMnO₂, LiNiO₂, LiMn₂O₄, Li(Ni_{1/2}Mn_{1/2})O₂, LiFePO₄, des polymères conducteurs, Li₄Ti₅O₁₂, des oxydes métalliques transitoires, TiO₂, SnO₂, Si et du soufre.

12. Procédé selon l'une quelconque des revendications 1 à 11, dans lequel les nanotubes de carbone sont chargés de catalyseurs ou modificateurs avant la fabrication des microtubes.

13. Procédé selon l'une quelconque des revendications 1 à 11, dans lequel les nanotubes de carbone sont chargés de catalyseurs ou modificateurs après ou au cours de la fabrication des microtubes.

14. Procédé selon l'une quelconque des revendications 1 à 13, incluant en outre l'étape de séchage en utilisant du vide, de l'air ou une atmosphère inerte.
